# EUROPEAN PATENT SPECIFICATION

(11) **EP 4 051 202 B1**
(45) Date of publication and mention of the grant of the patent: **09.04.2025**
(21) Application number: 20880402.1
(22) Date of filing: 28.10.2020
(51) Int. Cl.: A61F 13/15, A61F 13/49, A61F 13/515, A61F 13/534, A61F 13/539, A61L 15/58, B32B 5/02, B32B 5/26, C08L 23/14, C09J 123/14

(54) **TACKIFIER-FREE HOT MELT ADHESIVE COMPOSITION, METHOD FOR USING THE SAME, AND ARTICLE MADE USING THE SAME**
SCHMELZKLEBSTOFFZUSAMMENSETZUNG OHNE KLEBRIGMACHER, VERFAHREN ZU IHRER VERWENDUNG UND DAMIT HERGESTELLTER GEGENSTAND
COMPOSITION ADHÉSIVE THERMOFUSIBLE SANS AGENT POISSEUX, SA MÉTHODE D'UTILISATION ET ARTICLE FABRIQUÉ À L'AIDE DE CELLE-CI

(30) Priority: 31.10.2019 US 201962928504 P; 17.07.2020 US 202063053072 P
(43) Date of publication of application: 07.09.2022
(73) Proprietor: Bostik, Inc., Wauwatosa, WI 53226-3413 (US)
(72) Inventor: SECRIST, Kimberly, E., Wauwatosa, Wisconsin 53213 (US); JANETSKI, Neil, G., Milwaukee, Wisconsin 53208 (US); GRAY, Steven D., Mequon, Wisconsin 53092 (US)
(74) Representative: Arkema Patent
(86) International application number: PCT/US2020/057632
(87) International publication number: WO 2021/086896

(56) References cited:
- US-A1- 2005 096 613
- US-A1- 2015 024 649
- US-A1- 2015 351 977
- US-A1- 2017 165 133
- US-A1- 2017 165 133
- US-A1- 2019 233 686
- US-A1- 2019 233 686

## Description

### FIELD OF THE INVENTION

This invention relates to tackifier-free hot melt adhesive compositions suitable for use in a disposable hygiene article, such as diapers and training pants. The adhesive is especially well-suited for applications that require a high elongation at break, stress retention, and thermal stability.

### BACKGROUND OF THE INVENTION

Hot melt adhesives typically exist as a solid mass at ambient temperature and can be converted to a flowable liquid by the application of heat. These adhesives are particularly useful in manufacturing a variety of, typically, disposable goods where bonding of various substrates is often necessary. Specific applications include disposable diapers, hospital pads, feminine sanitary napkins, panty shields, surgical drapes and adult incontinent briefs, collectively known as disposable hygiene products or articles. Additionally, the adhesives may be used to make pre-formed, absorbent cores that are later inserted into a disposable or re-useable product. Other diversified applications have involved paper products, packaging materials, automotive headliners, appliances, tapes and labels. In most of these applications, the hot melt adhesive is heated to its molten state and then applied to a substrate, often named as the primary substrate. A second substrate, often named as the secondary substrate, is then immediately brought into contact with and compressed against the first. The adhesive solidifies on cooling to form a strong bond. The major advantage of hot melt adhesives is the absence of a liquid carrier, as would be in the case of water or solvent-based adhesives, thereby eliminating the costly process associated with solvent removal and potentially harmful and/or odorous solvents.

For many applications, hot melt adhesives are often extruded directly onto a substrate in the form of a thin film or a bead by using piston or gear pump equipment. The temperature of the die must be maintained well above the melting point of the adhesive to allow the molten hot melt material to flow through the application nozzle smoothly. For most applications, particularly those encountered in food packaging and disposable nonwovens hygienic article manufacturing, bonding of delicate and heat sensitive substrates, such as thin gauge plastic films, is often involved. This imposes an upper limit on coating temperature for hot melt adhesive applications. Today's commercial hot melts are typically formulated to have coating temperature below 200°C to avoid substrate burning or distortion. In this case, the adhesive is most typically sprayed from a nozzle at a set distance above the substrate or material of interest, such as a substrate containing super absorbent polymers. Several indirect or noncontact coating methods, through which a hot melt adhesive can be spray-coated with the aid of compressed air onto a substrate from a distance, have also been developed. These noncontact coating techniques include conventional spiral spray, Signature^{™}, Control Coat^{™}, UFD^{™}, and various forms of melt-blown methods. The indirect method, however, requires that the viscosity of the adhesives must be sufficiently low, usually in the range of 2,000 to 30,000 mPa·s, often in the range of 2,000 to 15,000 mPa·s, at the application temperature in order to obtain an acceptable coating pattern.

Hot melt adhesives are traditionally comprised of polymers, plasticizers, tackifying resins (also referred to herein as "tackifiers"), and optional additives such as waxes and anti-oxidants. Tackifiers have long been considered a necessary component of hot melt adhesives. Traditionally, the role of tackifiers with styrene-block-copolymer systems is to increase tack by increasing the glass transition temperature of the adhesive system. Tackifiers additionally help suppress the viscosity of the final formulation. However, tackifiers can be viewed negatively because they can contribute odor and volatile organic compounds (VOCs) to the adhesive formulation. Therefore, it is desired to make a hot melt adhesive free from tackifying resins. Such an adhesive would be desirably polyolefin-based since such adhesives handle high temperature well and are generally perceived as having lower odor than others, such as styrene block copolymer-based adhesives.

Efforts have been made to develop tackifier-free hot melt adhesives. For example, U.S. Patent No. 9,139,755 discloses a hot melt adhesive composition comprising about 10 to 90 wt.% of an amorphous polyolefin copolymer; about 50 to 70 wt.% 1-butene; about 10 to 90 wt.% of a heterophase polypropylene copolymer composition comprising propene and a comonomer comprising ethylene, 1-hexene or 1-octene and having amorphous character and crystalline blocks; and about 0.1 to 30 wt.% of a polyisobutylene plasticizer made with an AlCl₃; wherein the adhesive provides cohesive strength from the heterophase polypropylene copolymer and adhesive strength from the amorphous polyolefin copolymer.

In addition, U.S. Patent No. 8,623,480 discloses a hot melt adhesive composition comprising at least 55% by weight of a first polymer consisting of a non-functionalized amorphous poly alpha olefin polymer comprising greater than about 50% by weight polypropylene; a second polymer selected from the group consisting of polypropylene homopolymers, propylene copolymers, and combinations thereof; a functionalized polypropylene wax; and polyethylene wax. The hot melt adhesive composition is preferably tackifier-free.

International Patent Application No. WO 2013/039261 discloses a hot melt adhesive composition including: (A) a propylene homopolymer having a melting point of 100°C or lower which is obtainable by polymerizing propylene using a metallocene catalyst; and (B) an ethylene-based copolymer.

### SUMMARY OF THE INVENTION

There still exists a need in the art for an adhesive which has low tack and a sufficiently high elongation at break, stress retention, and thermal stability to be able to withstand significant swelling, such as the swelling of a superabsorbent polymer when wet, all the while still having a suitable viscosity at the desired application temperature.

Therefore, it would be advantageous to provide a hot melt adhesive that will overcome the shortcomings of the prior art adhesives mentioned above. In particular, it is desired to make a hot melt adhesive which does not contain any tackifiers. Such an adhesive is desirably polyolefin-based because such adhesives can handle high temperatures well and are generally perceived as having lower odor. Such an adhesive would have a sufficiently high elongation at break and stress retention to withstand the swelling of a superabsorbent polymer when wet or swollen/expanded. Such an adhesive would be particularly well suited as a micro-fiberized adhesive, responsible for containing superabsorbent polymers (SAP) within a hygiene article, such as a diaper, either alone or in conjunction with another adhesive and/or cellulose fibers. An adhesive serving this function is known to be providing core stabilization. The adhesive would not be required to have a high degree of tack, but is required to have very good elongation to contain the SAP as it swells/expands. A challenge in making a tackifier-free adhesive is developing a formulation with good thermal stability and "strong" enough to meet mechanical properties, such as elongation at break and stress retention.

In view of the shortcomings of the prior art, the present invention provides a hot melt adhesive composition comprising an essentially amorphous, single site-catalyzed, high molecular weight propylene copolymer; a semicrystalline, single site-catalyzed, low molecular weight propylene copolymer; polyisobutene; and a 1-butene-based copolymer polymer, wherein the 1-butene-based copolymer has a density of at most 0.907 g/cm³, wherein the adhesive is tackifier-free, the high molecular weight propylene copolymer has a weight average molecular weight of at least about 80,000 g/mol, preferably at least about 95,000 g/mol, and most preferably at least about 105,000 g/mol and has a heat of fusion of at most about 20 J/g, preferably at most about 15 J/g, and most preferably at most about 10 J/g; and the low molecular weight propylene copolymer has a weight average molecular weight of at most about 60,000 g/mol, preferably at most about 40,000 g/mol, and most preferably at most about 30,000 g/mol and has a heat of fusion of at least about 20 J/g, preferably at least about 25 J/g, and most preferably at least about 30 J/g. Such a composition provides both high elongation at break and stress retention while having exceptional thermal stability.

**In** accordance with an embodiment of the present invention, a method of making a laminate comprises the steps of: applying the hot melt adhesive composition of the invention in a molten state to a primary substrate; and mating a secondary substrate to the first substrate by contacting the secondary substrate with the adhesive composition.

**In** accordance with another embodiment of the present invention, an absorbent core comprises a first layer and a second layer, wherein at least one of the first layer and the second layer comprises superabsorbent polymers, and the first layer and the second layer are adhered to each other by a hot melt adhesive composition of the present invention and the adhesive composition adheres the superabsorbent polymers within the absorbent core.

In accordance with another embodiment of the present invention, a disposable hygiene article comprises the absorbent core of the invention.

### DETAILED DESCRIPTION OF THE INVENTION

In accordance with an embodiment of the present invention, a hot melt adhesive composition comprises (a) an amorphous, single site-catalyzed, high molecular weight propylene copolymer; (b) a semicrystalline, single site-catalyzed, low molecular weight propylene copolymer; (c) polyisobutene; and (d) a 1-butene-based copolymer polymer, wherein the 1-butene-based copolymer has a density of at most 0.907 g/cm³, wherein the composition is tackifier-free, the high molecular weight propylene copolymer has a weight average molecular weight of at least about 80,000 g/mol, preferably at least about 95,000 g/mol, and most preferably at least about 105,000 g/mol and has a heat of fusion of at most about 20 J/g, preferably at most about 15 J/g, and most preferably at most about 10 J/g; and the low molecular weight propylene copolymer has a weight average molecular weight of at most about 60,000 g/mol, preferably at most about 40,000 g/mol, and most preferably at most about 30,000 g/mol and has a heat of fusion of at least about 20 J/g, preferably at least about 25 J/g, and most preferably at least about 30 J/g. The adhesive composition may optionally comprise antioxidant(s), and optionally plasticizer(s). Such a composition provides both high elongation at break and stress retention while having exceptional thermal stability. According, such an adhesive is particularly well-suited to applications requiring the ability to elongate in use to a great extent (e.g., such as by 350% or more) and offer a high stress retention (such as 30% or more at 300% elongation after 10 minutes), and exceptional thermal stability (such as 90% viscosity retained at elevated temperatures after three days).

Both the first and second propylene copolymers used in this invention are single site-catalyzed. Single site catalyst systems (SSC) differ from the conventional catalysts (such as Ziegler-Natta catalysts) in at least one significant way; they have only a single active transition metal site for each catalyst molecule and the activity at this metal site is therefore identical for all the catalyst molecules. One type of SSC catalyst that has now been widely used on industrial scale is a metallocene catalyst system consisting of a catalyst and a co-catalyst or activator. The catalyst is a transition metal complex having a metal atom situated between two cyclic organic ligands; the ligands may be the same or different derivatives of cyclopentadiene. The co-catalyst can be any compound capable of activating the metallocene catalyst by converting a metallocene complex to a catalytically active species, and an example of such compound is alumoxane, preferably methylalumoxane having an average degree of oligomerization of from 4 - 30. For the purpose of this invention, other neutral or ionic activators can be used, including, but not limited to, various organic boron compounds such as tri(n-butyl)ammonium tetrakis(pentafluorophenyl borate, dimethylanilinium tetrakis(pentafluorophenyl borate, or trityl tetrakis(pentafluorophenyl borate. Another type of SSC catalyst is the constrained geometry catalyst (CGC).

As used herein, CGC refers to a sub-class of SSC catalyst system known as constrained geometry catalyst. Different from metallocenes, the constrained geometry catalyst (CGC) is characterized by having only one cyclic ligand linked to one of the other ligands on the same metal center in such a way that the angle at this metal between the centroid of the pi-system and the additional ligand is smaller than in comparable unbridged complexes. More specifically, the term CGC is used for ansa-bridged cyclopentadienyl amido complexes, though the definition goes far beyond this class of compounds. Hence, the term CGC is broadly used to refer to other more or less related ligand systems that may or may not be isolobal and/or isoelectronic with the ansa-bridged cyclopentadienyl amido ligand system. Furthermore, the term is frequently used for related complexes with long ansa-bridges that induce no strain.

Like metallocenes, suitable CGCs may be activated methylaluminoxane (MAO), perfluorinated boranes and trityl borates co-catalysts. The catalytic systems based on CGCs, however, display incorporation of higher alpha-olefins to a much larger extent than comparable metallocene based systems. Non-metallocene based SSCs, also referred to as post-metallocene, single-site catalysts for olefin polymerization are also known. Typical post-metallocene catalysts feature bulky, neutral, alpha-diimine ligands. These post-metallocene catalysts, however, are more frequently used for polymerization of ethylene to produce plastomers and elastomers. They are rarely used for polymerization of alpha-olefins, such as propylene. Single-site catalyst systems for olefin polymerization are well-known to those skilled in the art and are extensively discussed in two symposia entitled Stereoselective Polymerization with Single-Site Catalysts edited by Lisa S. Baugh and Jo Ann M. Canich published by CRC press (2008), and Polyolefins: 50 Years after Ziegler and Natta II: Polyolefins by Metallocenes and Other Single-Site Catalysts edited by Walter Kaminsky and published by Springer Heidelberg (2013).

The advancement of SSC catalyst systems herein discussed above has made it practical to produce propylene based polymers and copolymers having various chain microstructures and specific stereochemistry. Depending on the choice of catalyst and reaction conditions, specific types of propylene polymers and copolymers, for example, can be purposely made to have narrow molecular weight distribution, statistically random comonomer incorporation, high fraction of atactic chain sequences, and shorter crystallizable isotactic or syndiotactic chain sequences. Macroscopically, the polymers may exhibit relatively lower melting point, lower enthalpy of melting, lower crystallinity, and lower density and behave more similar to elastomers than to polypropylene made by conventional catalysts. Such polymers may have various weight average molecular weight (Mw) ranges, such as from 1,000 g/mol to 1,000,000 g/mol; a range of melting points, such as between 20°C to 150°C which is well below the melting point 170 °C of iPP; a range of enthalpies of melting, such as between 0 J/g and 100 J/g; and a range of densities, such as between 0.85 g/cc and 0.90 g/cc. Some of these polymers are well suited for hot melt adhesive applications.

Both the first and second propylene copolymers are comprised primarily of propylene units. This means that they comprise at least 50 weight percent of propylene. The two propylene copolymers can be polymers of propylene and the same co-mononer or different co-monomers. Preferably, the high molecular weight propylene copolymer is a copolymer of propylene and a co-monomer selected from the group consisting of ethylene and a C₄ - C₁₂ alkylene, preferably ethylene. Similarly, the low molecular weight propylene copolymer is a copolymer of propylene and a co-monomer selected from the group consisting of ethylene and a C₄ - C₁₂ alkylene, preferably ethylene. The first and second propylene copolymers may have the same or different co-monomer content. Preferably, the first and second propylene copolymers have an ethylene content of between about 5% and about 25%, more preferably between about 7% and about 20%, even more preferably between about 9% and about 17%, and most preferably between about 10% and about 15%.

The high molecular weight propylene copolymer has a weight average molecular weight of at least about 80,000 g/mol, preferably at least about 95,000 g/mol, and most preferably at least about 105,000 g/mol. Preferably, the weight average molecular weight of the high molecular weight propylene copolymer is at most about 250,000 g/mol, preferably at most about 200,000 g/mol, and most preferably at most about 175,000 g/mol. When upper and lower limits of ranges of a property or concentration of a constituent or of the adhesive or otherwise are set forth herein, any range extending from any lower limit to any upper limit is presumed to be contemplated by the present invention, as well as any range extending from any lower limit or any range extending from any upper limit. Therefore, the embodiments of the present invention include a high molecular weight propylene copolymer having a weight average molecular weight of at least about 80,000 g/mol to at most about 250,000 g/mol; a high molecular weight propylene copolymer having a weight average molecular weight of at least about 95,000 g/mol to at most about 250,000 g/mol; and a high molecular weight propylene copolymer having a weight average molecular weight of at least about 80,000 g/mol, as examples. The weight average molecular weight of the low molecular weight propylene copolymer is at most about 60,000 g/mol, preferably at most about 40,000 g/mol, and most preferably at most about 30,000 g/mol. Preferably, the weight average molecular weight of the low molecular weight propylene copolymer is at least about 10,000 g/mol, preferably at least about 15,000 g/mol, and most preferably at least about 17,500 g/mol. Weight average molecular weight of the two propylene copolymers set forth herein is characterized using a high temperature size exclusion chromatograph (SEC) using polypropylene reference standards.

Preferably, the molecular weights of the two propylene copolymers are significantly offset from one another. For example, in an embodiment of the invention, the molecular weight of the high molecular weight propylene copolymer is at least two times, preferably at least three times, and most preferably at least four times the molecular weight of the low molecular weight polypropylene copolymer.

The polydispersity indices (PDI) of the propylene copolymers may vary over a wide range and may be the same or different. The polydispersity indices of the two propylene polymers is preferably at most about 5, preferably at most about 4, and most preferably at most about 3. The polydispersity indices of the two propylene polymers is preferably at least about 1.5, preferably at least about 1.8, and most preferably at least about 2. These PDI values are generally characteristic of polymers that have been made using single site catalysts, such as metallocene catalysts. Preferably, the propylene copolymers of the present invention are made by using metallocene catalysts. The polydispersity indices (PDI) of the propylene copolymers are determined by dividing weight average molecular weight by number average molecular weight (M_{w}/Mₙ), with each value of Mw and Mn being determined by using data from the same analytical method (i.e., using a high temperature size exclusion chromatograph (SEC) using a polypropylene reference standard).

It has been found that the crystallinities of the two propylene copolymers are important to achieve certain purposes of the present invention. According to the invention, the high molecular weight propylene copolymer is essentially amorphous, meaning that it has relatively little, or no, crystallinity. Viewing crystallinity in terms of the polymer's heat of fusion, the high molecular weight propylene copolymer has a heat of fusion of at most about 20 J/g, preferably at most about 15 J/g, and most preferably at most about 10 J/g. Preferably, the high molecular weight propylene copolymer preferably has a heat of fusion of at least about 0 J/g, preferably at least about 2 J/g, and most preferably at least about 5 J/g. The low molecular weight propylene copolymer has a heat of fusion of at least about 20 J/g, preferably at least about 25 J/g, and most preferably at least about 30 J/g. Preferably, the low molecular weight propylene copolymer preferably has a heat of fusion of at most about 100 J/g, preferably at most about 75 J/g, and most preferably at least about 50 J/g. The test method used to determine these heat of fusion values is ASTM E793-01, "Standard Test Method for Enthalpies of Fusion and Crystallization by Differential Scanning Calorimetry."

Preferably, the heats of fusion of the two propylene copolymers are significantly offset from one another. For example, in an embodiment of the invention, the heat of fusion of the low molecular weight propylene copolymer is at least two times, preferably at least three times, and most preferably at least four times the heat of fusion of the high molecular weight polypropylene copolymer.

The melting temperatures, also referred to as the melting points, of the two propylene copolymers may vary over a wide range and may be the same or different. Preferably, the melting temperature of the low molecular weight propylene copolymer is between about 35°C and 100°C. More preferably, the melting temperature of the low molecular weight propylene copolymer is between about 40°C and about 90°C, even more preferably between about 50°C and about 80°C, and most preferably between about 55°C and about 75°C. Preferably, the melting temperature of the high molecular weight propylene copolymer is between about 70°C and about 130°C. More preferably, the melting temperature of the high molecular weight propylene copolymer is between about 75°C and about 125°C, even more preferably between about 85°C and about 115°C, and most preferably between about 90°C and about 110°C. The melting temperature as described herein is measured using Differential Scanning Calorimetry (DSC) according to ASTM E-794-01 except with one modification to the test in that a scanning temperature of 20°C per minute instead of 10°C per minute was used (the "DSC melting point").

The glass transition temperatures of the two propylene copolymers may also vary over a wide range and may be the same or different. Preferably, the glass transition temperatures of the each copolymer are between about -45°C and about -5°C. More preferably, the glass transition temperatures are between -35°C and -15°C, even more preferably between about -32°C and -20°C, and most preferably between about -30°C and -22°C. The glass transition temperature as described herein is measured using Differential Scanning Calorimetry (DSC) according to ASTM E-794-01 except with one modification to the test in that a scanning temperature of 20°C per minute instead of 10°C per minute was used.

The melt flow rate of the high molecular weight propylene copolymers may also vary over a wide range. The high molecular weight propylene copolymer may have a melt flow rate of from about 1 g/10 min to about 75 g/10 min, preferably from about 2 g/10 min to about 50 g/10 min, and most preferably from about 5 g/10 min to about 25 g/10 min. The low molecular weight propylene copolymer may have a melt flow rate, which would be higher than that of the high molecular weight propylene copolymer, or it may be too difficult to measure a melt flow rate. In one embodiment of the invention, a low molecular weight propylene copolymer is selected that has a viscosity of between about 500 cP to about 2,500 cP, preferably between about 750 to about 2,000 cP, and most preferably between about 1,000 to 1,500 cP, all at 190°C. As used herein, the melt flow rate of the two propylene copolymers is determined according to ASTM D 1238 using a 2.16 kilogram weight at 190°C.

Typical high molecular weight propylene copolymers suitable for use in the present invention include certain grades of VISTAMAXX^{™} propylene copolymers commercially available from ExxonMobile, including VISTAMAXX 6202 and 6502; and certain grades of VERSIFY^{™} propylene copolymers commercially available from The Dow Chemical Company, including VERSIFY 3000. Typical low molecular weight propylene copolymers suitable for use in the present invention include certain grades of VISTAMAXX^{™} propylene copolymers commercially available from ExxonMobile, including VISTAMAXX 8880.

In addition to two propylene copolymers, the adhesive composition of the present invention comprises polyisobutene. Herein, polyisobutene (PIB) refers to homopolymers or oligomers made from isobutene monomers and possessing the repeat unit of - [C(CH₃)₂CH₂]-. These materials may or may not contain some degree of unsaturation. Generally, they possess low number average molecular weights (950 - 4,500 g/mol) and can be thin or relatively viscous liquids at room temperature. According to embodiments of the present invention, the polyisobutene has a number average molecular weight of at least about 750 g/mol, preferably at least about 900 g/mol, more preferably at least about 1,500 g/mol, and most preferably at least about 2,000 g/mol. Preferably, the polyisobutene has a number average molecular weight of at most about 7,500 g/mol, preferably at most about 6,000 g/mol, and most preferably at most about 4,500 g/mol. They also typically possess a narrow polydispersity index (Mw/Mn < 4) though this is not critical to the present invention. Typical polyisobutenes suitable for use with the present invention include INDOPOL^{®} H-1900 or INDOPOL^{®} H-100, commercially available from Ineos Capital Ltd. Without being bound to any theory, it is believe that the polyisobutene serves to improve the cohesive strength of the adhesive and also serves to plasticize the propylene copolymers.

The hot melt adhesive composition of the present invention comprises a 1-butene-based copolymer. As used herein, the term "1-butene-based copolymer" means that the copolymer comprises greater than 50 mol % butene and is made of at least one other monomer in addition to butene. In one embodiment, the 1-butene-based copolymer selected may have a broad range of crystallinity (and a broad range of heat of fusion values). Preferably, the 1-butene-based copolymer comprises a butene-ethylene copolymer. In general, the weight average molecular weight of the 1-butene-based copolymer is preferably between about 15,000 and about 160,000 g/mol, and most preferably between about 30,000 and about 150,000 g/mol. As used herein, when referring to the weight average molecular weight of the 1-butene-based copolymer, the weight average molecular weight is determined by gel permeation chromatography using polypropylene standards. The 1-butene-based copolymer has a density of at most 0.907 g/cm³, preferably at most about 0.905 g/cm³, more preferably at most about 0.900 g/cm³, and most preferably at most about 0.895 g/cm³.

The hot melt adhesive composition of the present invention optionally comprises a plasticizer. The plasticizer may be any known compatible plasticizer and preferably is selected from the group consisting of mineral oil and synthetic poly-alphaolefin oils. A suitable plasticizer may also be selected from olefin oligomers and low molecular weight polymers, as well as vegetable and animal oils and derivatives thereof. The petroleum derived oils which may be employed are relatively high boiling materials containing only a minor proportion of aromatic hydrocarbons. In this regard, the aromatic hydrocarbons should preferably be less than 30% and more particularly less than 15% of the oil, as measured by the fraction of aromatic carbon atoms. More preferably, the oil may be essentially non-aromatic. The plasticizers that find usefulness in the present invention can be any number of different plasticizers but mineral oil and other plasticizers having an average molecular weight less than 5,000 daltons are particularly advantageous. As will be appreciated, plasticizers have typically been used to lower the viscosity of the overall adhesive composition without substantially decreasing the adhesive strength and/or the service temperature of the adhesive as well as to extend the open time and to improve flexibility of the adhesive.

Embodiments of the present invention are an adhesive based on mixtures of the high molecular weight propylene copolymer and the low molecular weight propylene copolymer in which the ratio of the high molecular weight propylene copolymer and the low molecular weight propylene copolymer is between about 1:1 and about 5:1, preferably between about 3:2 and about 4:1, more preferably between about 2:1 and about 7:2 and most preferably about 3:1.

The amounts of the various constituents may vary over a wide range, depending on the desired application temperature and other conditions and the desired performance characteristics of the adhesive. According to embodiments of the invention, the adhesive comprises:
the high molecular weight propylene copolymer in an amount of between about 10% and about 60% by weight, preferably between about 20% and about 50% by weight, and most preferably between about 25% and about 40% by weight, based on the total weight of the composition;
the low molecular weight propylene copolymer is present in an amount of between about 3% and about 40% by weight, preferably between about 4% and about 30% by weight, and most preferably between about 5% and about 20% by weight, based on the total weight of the composition; and
the polyisobutene is present in an amount of between about 5% and about 50% by weight, preferably between about 10% and about 40% by weight, and most preferably between about 15% and about 30% by weight, based on the total weight of the composition.

The invention includes any combination of any range of one constituent with any range or an unlimited amount of another constituent or both other constituents. The 1-butene-based copolymer may be present in an amount of between about 5% and about 40% by weight, preferably between about 7% and about 30% by weight, and most preferably between about 10% and about 25% by weight, based on the total weight of the composition. Also, when present, the plasticizer may be present in an amount of between about 5% and about 60% by weight, preferably between about 10% and about 50% by weight, and most preferably between about 15% and about 40% by weight, based on the total weight of the composition.

The present invention may optionally include an antioxidant, also referred to as a stabilizer. If included, the antioxidant may be present in an amount of from about 0.1% to about 3% by weight of the total adhesive composition. Preferably, from about 0.2% to 2% of an antioxidant is incorporated into the composition. The antioxidants which are useful in the hot melt adhesive compositions of the present invention are incorporated to help protect the polymers noted above, and thereby the total adhesive system, from the effects of thermal and oxidative degradation which normally occurs during the manufacture and application of the adhesive as well as in the ordinary exposure of the final product to the ambient environment. Among the applicable antioxidants are high molecular weight hindered phenols and multifunction phenols, such as sulfur and phosphorous-containing phenols. Hindered phenols are well-known to those skilled in the art and may be characterized as phenolic compounds that also contain sterically bulky radicals in close proximity to the phenolic hydroxyl group thereof. In particular, tertiary butyl groups generally are substituted onto the benzene ring in at least one of the ortho positions relative to the phenolic hydroxyl group. The presence of these sterically bulky substituted radicals in the vicinity of the hydroxyl group serves to retard its stretching frequency and correspondingly, its reactivity; this steric hindrance thus provides the phenolic compound with its stabilizing properties. Representative hindered phenols include: 1,3,5-trimethyl-2,4,6-tris(3-5-di-tert-butyl-4-hydroxybenzyl) benzene; pentaerythirtol tetrakis-3(3,5-di-tert-butyl-4-hydroxyphenyl) propionate; n-octadecyl-3(3,5-di-tert-butyl-4-hydroxyphenyl) propionate; 4,4'-methylenebis(4-methyl-6-tert butylphenol); 2,6-di-tert-butylphenol; 6-(4-hydroxyphenoxy)-2,4-bis(n-octylthio)-1,3,5-triazine; 2,3,6-tris(4-hydroxy-3,5-di-tert-butyl-phenoxy)-1,3,5-triazine; di-n-octadecyl-3,5-di-tert-butyl-4-hydroxybenzylphosphonate; 2-(n-octylthio)ethyl-3,5-di-tert-butyl-4-hydroxybenzoate; and sorbitol hexa-3(3,5-di-tert-butyl-4-hydroxy-phenyl) propionate.

The performance of these antioxidants may be further enhanced by utilizing, in conjunction therewith; (1) synergists such as, for example, thiodipropionate esters and phosphites; and (2) chelating agents and metal deactivators as, for example, ethylenediaminetetraacitic acid, slats thereof, and disalicylalpropylenediimine.

It should be understood that other auxiliary additives may be incorporated into the adhesive composition of the present invention in order to modify particular physical properties. These may include, for example, such materials as inert colorants e.g. titanium dioxide, fillers, fluorescent agents, UV absorbers, surfactants, other types of polymers, etc. Typical fillers include talc, calcium carbonate, clay silica, mica, wollastonite, feldspar, aluminum silicate, alumina, hydrated alumina, glass microspheres, ceramic microspheres, thermoplastic microspheres, baryte and wood flour. Surfactants are particularly important in hygienic disposable nonwoven products because they can dramatically reduce the surface tension, for example, of the adhesive applied to diaper core, thereby permitting quicker transport and subsequent absorption of urine by the core.

According to embodiments of the invention, waxes are included in the adhesive composition. Such waxes could include low molecular weight waxes, petroleum waxes such as paraffin wax, synthetic waxes, and polyolefin waxes. Preferably, the adhesive composition contains substantially no wax, such as less than 1% by weight, more preferably less than 0.5% by weight based on the total weight of the composition, and most preferably no wax.

According to embodiments of the invention, a hot melt adhesive composition consists essentially of, or consists of, an essentially amorphous, single site-catalyzed, high molecular weight propylene copolymer; a semicrystalline, single site-catalyzed, low molecular weight propylene copolymer; and polyisobutene and optionally a butene-rich copolymer and optionally, one or more of the other optional constituents mentioned herein, wherein the adhesive is tackifier-free. By being 'tackifier-free,' the composition contains no tackifiers. Classes of such tackifiers include: aliphatic and cycloaliphatic petroleum hydrocarbon resins; aromatic petroleum hydrocarbon resins and hydrogenated derivatives thereof; aliphatic/aromatic petroleum derived hydrocarbon resins and the hydrogenated derivatives thereof; aromatic modified cycloaliphatic resins and the hydrogenated derivatives thereof; polyterpene resins having a softening point of from about 10°C to about 140°C; copolymers and terpolymers of natural terpenes; natural and modified rosin; glycerol and pentaerythritol esters of natural and modified rosin; and phenolic-modified terpene resins. According to an embodiment of the invention, the adhesive composition does not contain an amorphous polyalpha olefin. According to another embodiment of the invention, the adhesive composition comprises less than 40% by weight, preferably less than 25% by weight, more preferably less than 10% by weight, still more preferably less than 5% by weight, and most preferably less than 1% by weight of an amorphous polyalpha olefin. According to another embodiment of the invention, the adhesive composition does not contain any styrene.

The flow characteristics and viscosity of the adhesive composition can be adjusted within certain parameters in ways known to one of ordinary skill in the art. The desired viscosity at a certain temperature will depend on the application conditions, including the manner of application, the desired flow upon application, the line speed, and the system used in such application, among other factors. According to embodiments of the invention, the viscosity of the composition is at most about 17,500 centipoise (cP) at 177°C (350°F), preferably at most about 15,000 centipoise (cP) at 177°C (350°F), and most preferably at most about 12,500 centipoise (cP) at 177°C (350°F). According to embodiments of the invention, the viscosity of the composition is at least about 1,000 centipoise (cP) at 177°C (350°F), preferably equal at least about 2,500 centipoise (cP) at 177°C (350°F), and most preferably at least about 5,000 centipoise (cP) at 177°C (350°F). The viscosity of the adhesive is measured according to ASTM D3236 with spindle 27.

Adhesives according to embodiments of the present invention exhibit excellent mechanical properties (e.g., high elongation at break and high stress retained) and thermal stability. Such properties make adhesives of the invention useful for hygiene, construction, and packaging applications, and especially suitable for core stabilization of absorbent cores containing superabsorbent polymers, which swell significantly upon being insulted with moisture. The hot melt composition of the present invention is further characterized by having elongation at break of at least about 350%, preferably at least about 400%, more preferably at least about 450%, and most preferably at least about 475%, when measured at a pull rate of 5.08 cm/min (2"/min). The inventive formulation further meets additional mechanical properties such as stress retained at 300% elongation after ten minutes. Preferably the stressed retained at 300% elongation after ten minutes is at least 30%, preferably at least about 40%, more preferably at least about 45%, and most preferably at least about 50%. In addition, embodiments of the present invention demonstrate excellent stress at yield, such as at least about 0.4 MPa, preferably at least about 0.5 MPa, and most preferably at least about 0.6 MPa. The hot melt adhesive composition further has exceptional thermal stability such that the viscosity retained at elevated temperatures is greater than about 85% over at least about three days, preferably greater than about 90% over at least about three days, and most preferably greater than about 90% over at least about five days. The methodologies for determining elongation at break, stress retained, stress at yield, and thermal stability (as reflected by viscosity retention) are set forth in more detail in the Examples section below.

The hot melt adhesive composition of the present invention may be formulated by using any of the mixing techniques known in the art. A representative example of the mixing procedure involves placing all the components in a jacketed mixing kettle equipped with a rotor, and thereafter raising the temperature of the mixture to a range from 150°C to 200°C to melt the contents. Any of the constituents may be pre-blended or added individually to the mixing kettle. For example, the polymers can be a preformed mixture or blend or can be added to the mixing kettle individually. It should be understood that the precise temperature to be used in this step would depend on the melting points of the particular ingredients. The mixing is allowed to continue until a consistent and uniform mixture is formed. The content of the kettle is protected with inert gas such as carbon dioxide or nitrogen during the entire mixing process. Without violating the spirit of the present invention, various additions and variations can be made to the procedure to produce the hot melt composition, such as, for example, applying vacuum to facilitate the removal of entrapped air. Other equipment useful for formulating the composition of the present invention includes, but not limited to, single or twin screw extruders or other variations of extrusion machinery, kneaders, intensive mixers, Ross^{™} mixers, and the like. The hot melt adhesive is then cooled to room temperature and formed into chubs with a protective skin formed thereon or into pellets for shipment and use.

The adhesive composition of the present invention may be used as a general purpose hot melt adhesive in a number of applications such as, for example, in disposable nonwoven hygiene articles, paper converting, flexible packaging, wood working, carton and case sealing, labeling and other assembly applications. Particularly preferred applications include nonwoven disposable diaper and feminine sanitary napkin construction, re-useable hygiene products that incorporate pre-formed cores, diaper and adult incontinent brief elastic attachment, diaper and napkin core stabilization, diaper backsheet lamination, industrial filter material conversion, surgical gown and surgical drape assembly, etc. Compositions of the present invention are especially suitable for use in core stabilization of absorbent cores having superabsorbent polymers for disposable hygiene articles, such as diapers, feminine care pads, and adult incontinence products.

The resulting hot melt adhesives may be applied to substrates using a variety application techniques. Examples includes hot melt glue gun, hot melt slot-die coating, hot melt wheel coating, hot melt roller coating, melt blown coating, spiral spray, contact or noncontact strand coatings branded as Signature^{™}, Control Coat^{™}, UFD^{™}, and the like. In a preferred embodiment, the hot melt adhesive is indirectly sprayed onto the substrates.

In an embodiment of the invention, a method of making a laminate comprises the steps of: (1) applying the hot melt adhesive composition of the invention in a molten state to a primary substrate; and (2) mating a secondary substrate to the primary substrate by contacting the secondary substrate with the adhesive composition. In an embodiment of the invention, the first substrate comprises a first layer (such as a bottom layer) of an absorbent core and the secondary substrate comprises a second layer (such as a top layer) of the absorbent core, wherein at least one of the first layer or the second layer have superabsorbent polymers associated therewith. The first substrate and secondary substrate may be a single continuous material, but folded over, so that the two folds form the first and secondary substrate.

Any suitable absorbent core having superabsorbent polymers may be used in connection with the present invention. Suitable absorbent cores are described in U.S. Patent Application Nos. 2017/0209616; 2017/0165133; and 2016/0270987. As described in U.S. Patent Application No. 2017/0165133, the absorbent core structure typically includes absorbent polymer material, such as hydrogel-forming polymer material, also referred to as absorbent gelling material, AGM, or super-absorbent polymer, SAP. This absorbent polymer material ensures that large amounts of bodily fluids, e.g. urine, can be absorbed by the absorbent article during its use and be locked away, thus providing low rewet and good skin dryness. Thinner absorbent core structures can be made by reducing or eliminating the traditional use of cellulose or cellulosic fibers in the absorbent core structure. To maintain the mechanical stability of these absorbent core structures, a fiberized net structure, which in some cases may be an adhesive, may be added to stabilize the absorbent polymer material. The absorbent core may also have additional adhesives, either to assist the fiberized net structure adhesive and/or to bond other core materials to each other and/or to other article components. The superabsorbent polymer material may be deposited on or associated with the first and second substrates and a fiberized net structure covers the superabsorbent polymer material on the respective first and second substrates.

In embodiments of the invention, the fiberized net structure comprises an adhesive composition of the present invention. The fiberized net structure may contain other materials, such as other adhesives or cellulose fibers. In another embodiment of the invention, the sole adhesive used in the fiberized net structure is an adhesive composition of the present invention. In still other embodiments of the invention, the fiberized net structure consists solely of one or more adhesive compositions of the present invention. In an embodiment of the invention, the first and second absorbent layers are combined together such that at least a portion of the fiberized net structure of the first absorbent layer contacts at least a portion of the fiberized net structure of the second absorbent layer and wherein an adhesive of the present invention used in the fiberized net structure serves to adhere to two layers together to form the absorbent core. In embodiments of the invention, both the first and second layers of the absorbent core have superabsorbent polymers associated therewith. In other embodiments of the invention, only one of the layers has superabsorbent polymers associated therewith.

In another embodiment of the method of making a laminate of the invention, the primary substrate is a first layer of an absorbent core and the secondary substrate is a super-absorbent polymer. The super-absorbent polymer may be deposited on the first layer before the application of the adhesive composition. In this embodiment, the adhesive may form a fiberized net over and around the superabsorbent polymer and may also adhere to the first layer. In a further embodiment, a second layer is formed in the same way and then the two layers are mated, before the adhesive is cooled, to form an absorbent core.

### EXAMPLES

The invention is further illustrated by way of the examples which are set forth below. To prepare the hot melt adhesive, all components were weighed into an unlined aluminum pint can and heated to 177°C while under a nitrogen blanket (5 scfh). A double-blade impellor in an over-head mixer was lowered into the aluminum can and agitated at 25 rpm until the polymers were moving sufficiently. Then, the speed was increased to 50 rpm until the mixture was homogenous and at a constant temperature. The formulation was deemed complete when the mix appeared homogenous and no clumps from polymer were visible. The formulation was then used to test viscosity, tensile properties, and/or to make laminates for end performance testing. The extreme compatibility of the system allowed for all components to go in together without compromising total mix time. This compatibility and stability created a very heat resistant system that was capable of maintaining viscosity when aged at 177°C for several days.

The ingredients listed below and in Tables 1 through 3 were used to make the adhesives. The numeric values listed for a given raw material are in weight percent and should equal to 100%.

Calsol 5500 is a naphthenic process oil available from Calumet Specialty Products.

Indopol H-1900 is a polyisobutene oligomer available with a number average molecular weight of 2,500 g/mol as determined by the supplier (Ineos Capital Ltd) using gel permeation chromatography (GPC), and all references to the number average molecular weight of the polyisobutene made herein are based on the same method.

Indopol H-100 is a polyisobutene oligomer available with a number average molecular weight of 910 g/mol as determined by the supplier (Ineos Capital Ltd) using gel permeation chromatography (GPC).

Irgafos 168 is a tris(2,4-di-tert-butylphenyl) phosphate available from BASF Chemicals and is used as an antioxidant.

Irganox 1010 is pentaerythritoltetrakis(3-(3,5-di-tert-butyl-4-hydroxyphenyl)propionate) available from BASF Corp. and is used as an antioxidant
Koattro PB M 1200M is a 1-butene copolymer with a density of 0.908 g/cm3 (tested by supplier according to ISO 1183-1) available from LyondellBasell Industries Holdings.

Koattro PB M 1500M is a 1-butene copolymer with a density of 0.890 g/cm3 (tested by supplier according to ISO 1183-1) available from LyondellBasell Industries Holdings.

Vistamaxx 6502, obtained from ExxonMobil Chemical Company, Houston, TX, is an essentially amorphous poly(propylene-co-ethylene) containing about 13 wt% of ethylene comonomer and having a weight average molecular weight (Mw) greater than 80,000 g/mol and a density (reported by supplier using ASTM D1505) of 0.865 g/cm³.

Vistamaxx 8380, obtained from Exxonmobil Chemical Company, Houston, TX, is a low molecular weight, low viscosity random propylene-ethylene copolymer. It has about 12 wt% of ethylene comonomer and a weight average molecular weight (Mw) less than 60,000 g/mol and a density (reported by supplier) of 0.864 g/cm³.

Vistamaxx 8880, obtained from Exxonmobil Chemical Company, Houston, TX, is a semicrystalline low molecular weight propylene copolymer consisting of about 6 wt% by weight of ethylene comonomer and having a weight average molecular weight (Mw) of less than 60,000 g/mol and a density (reported by supplier) of 0.879 g/cm³.

Viscosity was measured according to ASTM D3236 with Spindle 27 at 163, 177, and 190 °C. The spindle speed was adjusted so the percent torque was between 45% and 90%. The viscosity was to be low enough to yield fiber diameters between 10 - 60 µm when the adhesive is sprayed at 5 gsm with a Nordson Signature^{©} Low Flow nozzle.

Dogbones for tensile tests were made by pouring molten adhesive into silicone molds so that the total dogbone length was 7.62 cm x 2.54 cm (3.5"x 1.0") with a thickness, when flush with the mold, of 0.3175 cm (0.125"). The testing area of the dogbone was 1.27 cm x 1.27 cm (0.5"x0.5"). A hot spatula was used to scrape away any excess adhesive from the silicone mold so that the thickness of the dogbone was as close to 0.3175 cm (0.125") as possible. The sample was allowed to cool to room temperature for at least 12 hours before being tested for elongation to break, max stress, and other mechanical tests. The top and bottom of the dogbone were clamped into an Instron tensile tester so that only the 1.27 cm x 1.27 cm (0.5" x 0.5") testing area is exposed. The pull rate was 5.08 cm/min (2"/min) and was continued until the specimens broke. The elongation at break and max stress were automatically calculated in the BlueHill3 software available with Instron. The elongation at break was recorded as a percentage based on the difference of final length and the initial length divided by the initial length to determine if the adhesive can withstand the stretch from SAP particles swelling. A goal was to have the adhesive maintain about 475% elongation or greater. It was also deemed necessary to have good stress retention over time when held at a given elongation. The elongation value was 300%; meaning a 1.27 cm (0.5") initial test height would stretch (at 5.08 cm/min (2"/min)) until the jaws on the Instron were a total distance of 5.08 cm (2") apart. The stress or load value is recorded upon reaching 300 %. Then the specimen is held at 300% elongation for ten minutes before a final stress or load value is noted. The "Stress Retained at 300 % elongation, 10 min (%)" as found in the below tables refers to the stress after the ten minute hold at 300% elongation divided by the initial stress at 300% elongation times 100, so the value is listed as a percentage of stress maintained. This factor should be at least 30%, preferably at least about 40%, and most preferably at least about 45% or even at least about 50%.

Pattern quality was determined qualitatively and quantitatively. The adhesive was heated to approximately 190°C and pumped through hoses to a Signature Low Flow Nozzle (Nordson Corp.) at 5 gsm coat-weight with a line speed of 60.96 m/min (200 ft/min). Air-flow was adjusted to create the most visually fiberized-looking pattern with minimal agglomerates (those having a size about more than double the average size of droplet) or fly-away adhesive strands (qualitative). On average, air pressure was around 20 psi, but this is dependent on the set-up of each piece of equipment and is only intended as a reference. The nozzle head was positioned 20 mm above the primary substrate, which was a 33 gsm spunbond nonwoven. The secondary substrate was a release liner so the samples could be better analyzed. Qualitative analysis was done with a microscope to determine the diameter of the fibers produced during the spray application. The goal was to produce fiber diameters less than 60 µm and preferably less than 30 µm. The adhesive's tensile properties are a function of the fiber diameter, so the desired fiber diameter may change depending on the adhesive formulation, the desired properties, and other conditions.

Thermal Stability was determined by pouring approximately 150 g of adhesive into an 8 oz glass jar and loosely securing the lid. The jar was placed in an oven at elevated temperature, namely 177 °C. The jar was removed from the oven each day to look for charring or gelling that would suggest incompatibility. Additionally, a 10 g slug of adhesive was poured from the jar each day to determine viscosity retention. Viscosity retention is calculated by dividing the viscosity at a given temperature by the initial viscosity before heat aging and multiplying by 100. Preferably, the viscosity retained would be greater than 85 % over at least three days. Ideally, the viscosity retained would be greater than 90% over at least three days or even at least five days.

**Table 1. Importance of polyisobutene (PIB)**

| | EX. 1 | EX.2 | EX.3 | CE.1 |
|---|---|---|---|---|
| Indopol H-1900, wt% | 20.27 | 24.35 | - | - |
| Indopol H-100, wt% | - | - | 20.27 | - |
| Calsol 5550, wt% | 22.15 | 26.62 | 22.15 | 42.42 |
| Vistamaxx 6502, wt% | 29.17 | 35.05 | 29.17 | 29.17 |
| Vistamaxx 8880, wt% | 10.97 | 13.18 | 10.97 | 10.97 |
| Koattro PB 1500M, wt% | 16.64 | - | 16.64 | 16.64 |
| IRGAFOS 168, wt% | 0.53 | 0.53 | 0.53 | 0.53 |
| IRGANOX 1010, wt% | 0.27 | 0.27 | 0.27 | 0.27 |
| Viscosity at 177°C (350°F) (cP) | 11,300 | 13,300 | 9,638 | 7,325 |
| Viscosity at 191°C (375°F) (cP) | 8,300 | - | 6,950 | 5,313 |
| Elongation at break (%) | 669 | 536 | 463 | 393 |
| Max stress (MPa) | 0.68 | 0.74 | 0.61 | 0.55 |
| Stress Retained at 300 % elongation, 10 min (%) | 54.1 | 49.6 | 34.7 | 0.0 |

Table 1 demonstrates the importance of the polyisobutene (PIB) component and the optional butene-rich polymer. EX. 2 shows that the butene-rich copolymer is optional, but preferred to include as it lowers the viscosity, shown in EX. 1 and increases the elongation and stress retained. In comparative example one (CE1), the use of only a naphthenic oil results in an adhesive that displays an unacceptably low elongation at break that consequently failed during the hold at 300 % elongation. Inventive examples 1 and 2 containing PIB had an elongation at break greater than 525% and retained greater than about 50% of their original stress after 10 minutes at 300% elongation. Example 3 containing the lower molecular weight PIB resulted in about 35% stress retained at 300% elongation after 10 minutes and 463% elongation at break. All examples of the invention had a maximum stress of at least 0.61 MPa. Furthermore, Examples 1 and 2 desirably produced an average fiber diameter of 17µm when 5gsm was applied at 190°C.

**Table 2. Importance of high molecular weight propylene copolymer compound**

| | | CE. 3 |
|---|---|---|
| Indopol H-1900, wt% | | 20.27 |
| Calsol 5550, wt% | | 22.15 |
| Koattro PB 1500M, wt% | | 16.64 |
| Irgafos 168, wt% | | 0.53 |
| Irganox 1010, wt% | | 0.27 |
| Vistamaxx 6502, wt% | | - |
| Vistamaxx 8880, wt% | | 10.97 |
| Vistamaxx 8380, wt% | | 29.17 |
| Viscosity at 177°C (350°F) (cP) | | 1063 |
| Viscosity at 191°C (375°F) (cP) | | 793 |
| Elongation at break (%) | | 115 |
| Max stress (MPa) | | 0.18 |
| Stress Retained at 300 % elongation, 10 min (%) | | 0.0 |

Table 2 demonstrates the role of the high molecular weight propylene copolymer component. Comparative example three (CE3) replaces the high molecular weight component with an additional low molecular weight component. The loss of a high molecular weight component results in a drastic decrease in elongation at break and a complete loss of stress retained at 300% elongation after 10 minutes.

**Table 3. Impact of third polymer having too high specific gravity**

| | CE. 4 |
|---|---|
| Calsol 5550, wt% | 22.18 |
| Indopol H-1900, wt% | 20.29 |
| Vistamaxx 6502, wt% | 29.21 |
| Vistamaxx 8880, wt% | 10.98 |
| KOATTRO PB M 1200M, wt% | 16.66 |
| Irgafos 168, wt% | 0.53 |
| Irganox 1010, wt% | 0.27 |
| Viscosity at 163°C (325 °F) (cP) | 23,650 |
| Elongation at break (%) | 380 |
| Max stress (MPa) | 0.85 |
| Stress Retained at 300 % elongation, 10 min (%) | 0.0 |

Comparative example four (CE4) contains a butene-rich copolymer with a specific gravity of 0.908 g/cm³. The resulting formulation becomes partially incompatible resulting in a quick decrease in elongation at break and complete loss of stress retained at 300% elongation after ten minutes.

**Table 4. Thermal Stability-Viscosity Retention at 177°C**

| Duration at 177°C (days) | EX. 1 (%) | CE. 5* (%) |
|---|---|---|
| 1 | 94.6 | 86.3 |
| 2 | 94.5 | 70.7 |
| 3 | 94.3 | 51.8 |
| 4 | 93.8 | 34.8 |
| 5 | 92.6 | 24.9 |

| | | |
|---|---|---|
| *CE. 5 is a commercially available styrene block copolymer available from Bostik Inc. | | |

Table 4 demonstrates the exceptional thermal stability via viscosity retention when aged in an oven at 177°C. Ideally, hot melt adhesives are heated only for the duration of time needed, but occasionally they can remain in the molten state for several hours to several days. For example, if a production line is having issues, the adhesive could be retained in a molten state before the line is running again. Additionally, some manufacturers do not produce products over weekends and occasionally may leave the melt tank on to avoid having to wait for adhesive to re-melt upon re-starting a line. Therefore, it is advantageous to have an adhesive with exceptional thermal stability to allow for these circumstances. Preferably, the hot melt composition would have a retained viscosity at elevated temperatures greater than about 85% over at least about three days, preferably the greater than about 90% over at least about three days, and most preferably greater than about 90% over at least about five days.

Unless defined otherwise, all technical and scientific terms used herein have the same meanings as commonly understood by one of ordinary skill in the art to which this invention belongs. All references cited in this specification are to be taken as indicative of the level of skill in the art. Nothing herein is to be construed as an admission that the invention is not entitled to antedate such disclosure by virtue or prior invention.

## Claims

1. A hot melt adhesive composition comprising:
(a) an essentially amorphous, single site-catalyzed, high molecular weight propylene copolymer;
(b) a semicrystalline, single site-catalyzed, low molecular weight propylene copolymer;
(c) polyisobutene; and
(d) a 1-butene-based copolymer polymer, wherein the 1-butene-based copolymer has a density of at most 0.907 g/cm³;
wherein:
the composition is tackifier-free,
the high molecular weight propylene copolymer has a weight average molecular weight of at least about 80,000 g/mol, preferably at least about 95,000 g/mol, and most preferably at least about 105,000 g/mol and has a heat of fusion of at most about 20 J/g, preferably at most about 15 J/g, and most preferably at most about 10 J/g; and
the low molecular weight propylene copolymer has a weight average molecular weight of at most about 60,000 g/mol, preferably at most about 40,000 g/mol, and most preferably at most about 30,000 g/mol and has a heat of fusion of at least about 20 J/g, preferably at least about 25 J/g, and most preferably at least about 30 J/g.

2. The composition of claim 1, wherein:
the high molecular weight propylene copolymer has a polydispersity index of less than about 5, preferably less than about 4, and most preferably less than about 3; and
the low molecular weight propylene copolymer has a polydispersity index of less than about 5, preferably less than about 4, and most preferably less than about 3.

3. The composition of claim 1, wherein:
the high molecular weight propylene copolymer is a copolymer of propylene and a co-monomer selected from the group consisting of ethylene and a C₄ - C₁₂ alkylene, preferably ethylene; and
the low molecular weight propylene copolymer is a copolymer of propylene and a co-monomer selected from the group consisting of ethylene and a C₄ - C₁₂ alkylene, preferably ethylene.

4. The composition of claim 1, wherein the polyisobutene has a number average molecular weight of at least about 750 g/mol, preferably at least about 900 g/mol, more preferably at least about 1,500 g/mol, and most preferably at least about 2,000 g/mol.

5. The composition of claim 1, wherein
the high molecular weight propylene copolymer is present in an amount of between about 10% and about 60% by weight, preferably between about 20% and about 50% by weight, and most preferably between about 25% and about 40% by weight, based on the total weight of the composition;
the low molecular weight propylene copolymer is present in an amount of between about 3% and about 40% by weight, preferably between about 4% and about 30% by weight, and most preferably between about 5% and about 20% by weight, based on the total weight of the composition; and
the polyisobutene is present in an amount of between about 5% and about 50% by weight, preferably between about 10% and about 40% by weight, and most preferably between about 15% and about 30% by weight, based on the total weight of the composition.

6. The composition of claim 1, wherein the 1-butene-based copolymer comprises a butene-ethylene copolymer.

7. The composition of claim 1 further comprising a plasticizer, the plasticizer being preferably selected from the group consisting of a mineral oil and a synthetic poly-alphaolefin oil and mixtures thereof.

8. The composition of claim 1, wherein the composition does not contain an amorphous polyalpha olefin.

9. The composition of claim 1, wherein the composition does not contain a wax.

10. The composition of claim 1, wherein the molecular weight of the high molecular weight propylene copolymer is at least two times, preferably at least three times, and most preferably at least four times the molecular weight of the low molecular weight propylene copolymer.

11. The composition of claim 1 further comprising an antioxidant.

12. A method of making a laminate comprising the steps of:
applying the hot melt adhesive composition of any of claims 1 to 11 in a molten state to a primary substrate; and
mating a secondary substrate to the primary substrate by contacting the secondary substrate with the adhesive composition.

13. An absorbent core comprising a first layer and a second layer, wherein at least one of the first layer and the second layer comprises superabsorbent polymers, and the first layer and the second layer are adhered to each other by a hot melt adhesive composition of any of claims 1 to 11 and the adhesive composition adheres the superabsorbent polymers within the absorbent core.

14. A disposable hygiene article comprising the absorbent core of claim 13.

## Patentansprüche

1. Schmelzklebstoffzusammensetzung, umfassend:
(a) ein im Wesentlichen amorphes, Single-Site-katalysiertes, hochmolekulares Propylen-Copolymer;
(b) ein teilkristallines, Single-Site-katalysiertes, niedermolekulares Propylen-Copolymer;
(c) Polyisobuten; und
(d) ein Copolymer-Polymer auf Basis von 1-Buten, wobei das Copolymer auf Basis von 1-Buten eine Dichte von höchstens 0,907 g/cm³ aufweist;
wobei:
die Zusammensetzung klebrigmacherfrei ist,
das hochmolekulare Propylen-Copolymer ein gewichtsmittleres Molekulargewicht von mindestens etwa 80.000 g/mol, vorzugsweise mindestens etwa 95.000 g/mol und ganz besonders bevorzugt mindestens etwa 105.000 g/mol aufweist und eine Schmelzwärme von höchstens etwa 20 J/g, vorzugsweise höchstens etwa 15 J/g und ganz besonders bevorzugt höchstens etwa 10 J/g aufweist; und
das niedermolekulare Propylen-Copolymer ein gewichtsmittleres Molekulargewicht von höchstens etwa 60.000 g/mol, vorzugsweise höchstens etwa 40.000 g/mol und ganz besonders bevorzugt höchstens etwa 30.000 g/mol aufweist und eine Schmelzwärme von mindestens etwa 20 J/g, vorzugsweise mindestens etwa 25 J/g und ganz besonders bevorzugt mindestens etwa 30 J/g aufweist; und

2. Zusammensetzung nach Anspruch 1, wobei:
das hochmolekulare Propylen-Copolymer einen Polydispersitätsindex von weniger als etwa 5, vorzugsweise weniger als etwa 4 und ganz besonders bevorzugt weniger als etwa 3 aufweist; und
das niedermolekularen Propylen-Copolymer einen Polydispersitätsindex von weniger als etwa 5, vorzugsweise weniger als etwa 4 und ganz besonders bevorzugt weniger als etwa 3 aufweist.

3. Zusammensetzung nach Anspruch 1, wobei:
es sich bei dem hochmolekularen Propylen-Copolymer um ein Copolymer von Propylen und einem Comonomer, das aus der Gruppe bestehend aus Ethylen und einem C₄-C₁₂-Alkylen ausgewählt ist, vorzugsweise Ethylen, handelt; und
es sich bei dem niedermolekularen Propylen-Copolymer um ein Copolymer von Propylen und einem Comonomer, das aus der Gruppe bestehend aus Ethylen und einem C₄-C₁₂-Alkylen ausgewählt ist, vorzugsweise Ethylen, handelt.

4. Zusammensetzung nach Anspruch 1, wobei das Polyisobuten ein zahlenmittleres Molekulargewicht von mindestens etwa 750 g/mol, vorzugsweise mindestens etwa 900 g/mol, weiter bevorzugt mindestens etwa 1500 g/mol und ganz besonders bevorzugt mindestens etwa 2000 g/mol aufweist.

5. Zusammensetzung nach Anspruch 1, wobei
das hochmolekulare Propylen-Copolymer in einer Menge zwischen etwa 10 und etwa 60 Gew.-%, vorzugsweise zwischen etwa 20 und etwa 50 Gew.-% und ganz besonders bevorzugt zwischen etwa 25 und etwa 40 Gew.-%, bezogen auf das Gesamtgewicht der Zusammensetzung, vorliegt;
das niedermolekulare Propylen-Copolymer in einer Menge zwischen etwa 3 und etwa 40 Gew.-%, vorzugsweise zwischen etwa 4 und etwa 30 Gew.-% und ganz besonders bevorzugt zwischen etwa 5 und etwa 20 Gew.-%, bezogen auf das Gesamtgewicht der Zusammensetzung, vorliegt; und
das Polyisobuten in einer Menge zwischen etwa 5 und etwa 50 Gew.-%, vorzugsweise zwischen etwa 10 und etwa 40 Gew.-% und ganz besonders bevorzugt zwischen etwa 15 und etwa 30 Gew.-%, bezogen auf das Gesamtgewicht der Zusammensetzung, vorliegt.

6. Zusammensetzung nach Anspruch 1, wobei das Copolymer auf Basis von 1-Buten ein Buten-Ethylen-Copolymer umfasst.

7. Zusammensetzung nach Anspruch 1, ferner umfassend einen Weichmacher, wobei der Weichmacher vorzugsweise aus der Gruppe bestehend aus einem Mineralöl und einem synthetischen Polyalphaolefin-Öl und Mischungen davon ausgewählt ist.

8. Zusammensetzung nach Anspruch 1, wobei die Zusammensetzung kein amorphes Polyalphaolefin enthält.

9. Zusammensetzung nach Anspruch 1, wobei die Zusammensetzung kein Wachs enthält.

10. Zusammensetzung nach Anspruch 1, wobei das Molekulargewicht des hochmolekularen Propylen-Copolymers mindestens das Zweifache, vorzugsweise mindestens das Dreifache und ganz besonders bevorzugt mindestens das Vierfache des Molekulargewichts des niedermolekularen Propylen-Copolymers beträgt.

11. Zusammensetzung nach Anspruch 1, ferner umfassend ein Antioxidans.

12. Verfahren zur Herstellung eines Laminats, das folgende Schritte umfasst:
Aufbringen der Schmelzklebstoffzusammensetzung nach einem der Ansprüche 1 bis 11 in schmelzflüssigem Zustand auf ein primäres Substrat; und
Zusammenfügen eines sekundären Substrats mit dem ersten Substrat durch Inkontaktbringen des sekundären Substrats mit der Klebstoffzusammensetzung.

13. Absorbierender Kern, umfassend eine erste Schicht und eine zweite Schicht, wobei die erste Schicht und/oder die zweite Schicht superabsorbierende Polymere umfassen und die erste Schicht und die zweite Schicht mit einer Schmelzklebstoffzusammensetzung nach einem der Ansprüche 1 bis 11 verklebt sind und die Klebstoffzusammensetzung die superabsorbierenden Polymere in dem absorbierenden Kern verklebt.

14. Einweg-Hygieneartikel, umfassend den absorbierenden Kern nach Anspruch 13.

## Revendications

1. Composition d'adhésif thermofusible comprenant :
(a) un copolymère de propylène de poids moléculaire élevé, essentiellement amorphe, catalysé sur un seul site ;
(b) un copolymère de propylène semi-cristallin de faible poids moléculaire, catalysé sur un seul site, ;
(c) un polyisobutène ; et
(d) un polymère copolymère à base de 1-butène, dans laquelle le copolymère à base de 1-butène a une densité d'au plus 0,907 g/cm³ ;
dans laquelle :
la composition est exempte d'agent tackifiant,
le copolymère de propylène de poids moléculaire élevé possède un poids moléculaire moyen en poids d'au moins environ 80 000 g/mole, de préférence d'au moins environ 95 000 g/mole, et le plus préférablement d'au moins environ 105 000 g/mole, et possède une chaleur de fusion d'au plus environ 20 J/g, de préférence d'au plus environ 15 J/g, et le plus préférablement d'au plus environ 10 J/g ; et
le copolymère de propylène de faible poids moléculaire a un poids moléculaire moyen en poids d'au plus environ 60 000 g/mole, de préférence d'au plus environ 40 000 g/mole, et le plus préférablement d'au plus environ 30 000 g/mole et a une chaleur de fusion d'au moins environ 20 J/g, de préférence d'au moins environ 25 J/g, et le plus préférablement d'au moins environ 30 J/g.

2. Composition selon la revendication 1, dans laquelle :
le copolymère de propylène de poids moléculaire élevé présente un indice de polydispersité inférieur à environ 5, de préférence inférieur à environ 4, et le plus préférablement inférieur à environ 3 ; et
le copolymère de propylène de faible poids moléculaire présente un indice de polydispersité inférieur à environ 5, de préférence inférieur à environ 4, et le plus préférablement inférieur à environ 3.

3. Composition selon la revendication 1, dans laquelle :
le copolymère de propylène de poids moléculaire élevé est un copolymère de propylène et d'un co-monomère sélectionné dans le groupe constitué par l'éthylène et un alkylène en C₄ - C₁₂, de préférence l'éthylène ; et
le copolymère de propylène de faible poids moléculaire est un copolymère de propylène et d'un co-monomère choisi dans le groupe constitué par l'éthylène et un alkylène en C₄ - C₁₂, de préférence l'éthylène.

4. Composition selon la revendication 1, dans laquelle le polyisobutène a un poids moléculaire moyen en nombre d'au moins environ 750 g/mole, de préférence d'au moins environ 900 g/mole, mieux encore d'au moins environ 1 500 g/mole, et le plus préférablement d'au moins environ 2 000 g/mole.

5. Composition selon la revendication 1, dans laquelle le copolymère de propylène de poids moléculaire élevé est présent en une quantité comprise entre environ 10 % et environ 60 % en poids, de préférence entre environ 20 % et environ 50 % en poids, et mieux encore entre environ 25 % et environ 40 % en poids, par rapport au poids total de la composition ;
le copolymère de propylène de faible poids moléculaire est présent en une quantité comprise entre environ 3 % et environ 40 % en poids, de préférence entre environ 4
% et environ 30 % en poids, et le plus préférablement entre environ 5 % et environ 20 % en poids, par rapport au poids total de la composition ; et
le polyisobutène est présent en une quantité comprise entre environ 5 % et environ 50 % en poids, de préférence entre environ 10 % et environ 40 % en poids, et le plus préférablement entre environ 15 % et environ 30 % en poids, par rapport au poids total de la composition.

6. Composition selon la revendication 1, dans laquelle le copolymère à base de 1-butène comprend un copolymère de butène-éthylène.

7. Composition selon la revendication 1, comprenant en outre un plastifiant, le plastifiant étant de préférence choisi dans le groupe constitué par une huile minérale et une huile de poly-alphaoléfine synthétique et leurs mélanges.

8. Composition selon la revendication 1, dans laquelle la composition ne contient pas de polyalphaoléfine amorphe.

9. Composition selon la revendication 1, dans laquelle la composition ne contient pas une cire.

10. Composition selon la revendication 1, dans laquelle le poids moléculaire du copolymère de propylène de poids moléculaire élevé est au moins deux fois, de préférence au moins trois fois, et le plus préférablement au moins quatre fois le poids moléculaire du copolymère de propylène de poids moléculaire faible.

11. Composition selon la revendication 1, comprenant en outre un antioxydant.

12. Procédé de fabrication d'un stratifié comprenant les étapes de :
application de la composition d'adhésif thermofusible selon l'une quelconque des revendications 1 à 11 à l'état fondu sur un substrat primaire ; et
accouplement d'un substrat secondaire avec le substrat primaire par mise en contact du substrat secondaire avec la composition d'adhésif.

13. Noyau absorbant comprenant une première couche et une seconde couche, dans lequel au moins l'une parmi la première couche et la seconde couche comprend des polymères superabsorbants, et la première couche et la seconde couche sont collées l'une avec l'autre par une composition d'adhésif thermofusible selon l'une quelconque des revendications 1 à 11 et la composition d'adhésif adhère aux polymères superabsorbants à l'intérieur du noyau absorbant.

14. Article d'hygiène jetable comprenant le noyau absorbant selon la revendication 13.
